# EUROPEAN PATENT APPLICATION

(11) **EP 1 400 912 A2**
(43) Date of publication of application: **24.03.2004**
(21) Application number: 03255703.5
(22) Date of filing: 12.09.2003
(51) Int. Cl.: G06F 19/00

(54) **Information processing method and system using medical examination device as medium**

(30) Priority: 12.09.2002 JP 2002267327
(71) Applicant: CANON KABUSHIKI KAISHA, Tokyo (JP)
(72) Inventor: Ogura, Masaya, Ohta-ku, Tokyo (JP)
(74) Representative: Beresford, Keith Denis Lewis

(57) **Abstract**

An information processing method that utilizes a medical examination device as a medium, which has been assigned a unique identification used for medical examinations and diagnoses, and a memory into which particular additional information about the medical examination device is remotely writable through a network based on the identification of the medical examination device includes the steps of identifying the identification of the medical examination device, and writing down in the memory the particular additional information relating to a usage of the medical examination device while correlating the particular additional information with the identification, and sharing and utilizing the particular additional information about the medical examination device among a plurality of users based on the identification.

## Description

The present invention relates generally to information processing method and system using a medical examination device (MED) as a medium, and more particularly to those in a medical system that uses a MED, such as a DNA chip, a lab on a chip, a micro-array, a micro-TAS, a device for inspection using a quarts crystal microbalance (QCM) reaction, etc. for medical examinations, diagnoses, etc.

Recent medical examinations, diagnoses, etc. have actively developed a medical system that uses a MED referred to as a QCM DNA chip, a lab on a chip, a micro-array, a micro-TAS, etc.

However, widespread MEDs have raised a issue of managements of the MEDs and information obtained from the MEDs. For example, it is known that the MED has a short product life cycle, and requires careful control. In addition, usages are different according to MED types, and a misuse results in a different result, causing an improper diagnosis. The misdiagnosis is a great problem in a medical inspection result.

It is conceivable that mailing is used to inform a result obtained from a chip, but this procedure is arduously repeated by the number of institutions when plural institutions need the result. It is quite important to securely process information of an MED that may identify personal information.

There may be recited following considerations between institutions handling MEDs and individual users:
(1) MED vendors (who manufacture and ship MEDs) need control MEDs' product life cycles, circulations and purchasers.
(2) Distributors (who circulate and sell MEDs) need confirm shipping dates of MEDs.
(3) Medical institutions and drugstores (which supply and sell MEDs) need to use MEDs within their lifetimes, to confirm their contents, to recognize their usages and inform them to individual users, and to recognize inspection results of MEDs.
(4) Individual users or patients need to recognize usages and inspection results of MEDs.
(5) Inspection institutions (which inspect MEDs and supply inspection results) need to recognize usage records of MEDs, and to easily inform inspection results of MEDs.
(6) MED vendors (who recycle MEDs) need to recognize usage records of MEDs, and easily inform recycles of MEDs.

As apparent from the above, institutions handling MEDs and individual users have common and specific considerations regarding managements of MEDs themselves and information obtained from the MEDs. However, no approaches have not yet been developed for sharing and effectively utilizing information obtained from MEDs among institutions handling MEDs and individual users.

Accordingly, it is an exemplary object of the present invention to provide information processing method and system using a medical examination device as a medium, which enables information obtained from the medical examination devices to be shared and effectively utilized among institutions handling medical examination devices and individual users.

An information processing method of one aspect according to the present invention that utilizes a medical examination device as a medium, which has been assigned a unique identification used for medical examinations and diagnoses, and a memory into which particular additional information about the medical examination device is remotely writable through a network based on the identification of the medical examination device includes the steps of identifying the identification of the medical examination device, and writing down in the memory the particular additional information relating to a usage of the medical examination device while correlating the particular additional information with the identification, and sharing and utilizing the particular additional information about the medical examination device among a plurality of users based on the identification.

An information processing method of another aspect according to the present invention that utilizes a medical examination device as a medium, which has been assigned a unique identification used for medical examinations and diagnoses, and a memory into which particular additional information about the medical examination device is remotely writable through a network based on the identification of the medical examination device includes the steps of identifying the identification of the medical examination device, and writing down in the memory first particular additional information relating to a usage of the medical examination device while correlating the first particular additional information with the identification, writing down second particular additional information in the memory while correlating the second particular additional information with the identification, reading out one or more pieces from among the first and second particular additional information based on the identification, and sharing and utilizing plural pieces of particular additional information about the medical examination device among a plurality of users based on the identification.

An information processing method of still another aspect according to the present invention that utilizes a medical examination device as a medium, which has been assigned a unique identification used for medical examinations and diagnoses, and a memory into which particular additional information about the medical examination device is remotely writable through a network based on the identification of the medical examination device includes the steps of identifying the identification of the medical examination device, and writing down in the memory first particular additional information relating to a usage of the medical examination device while correlating the first particular additional information with the identification, writing down second particular additional information relating to an inspection in the memory while correlating the second particular additional information with the identification, writing down third particular additional information in the memory while correlating the third particular additional information with the identification, reading out one or more pieces from among the first to third particular additional information based on the identification, and sharing and utilizing plural pieces of particular additional information about the medical examination device among a plurality of users based on the identification.

An information processing method of another aspect according to the present invention that utilizes a medical examination device as a medium, which has been assigned a unique identification used for medical examinations and diagnoses, and a memory into which particular additional information about the medical examination device is remotely writable through a network based on the identification of the medical examination device includes the steps of identifying the identification of the medical examination device, and writing down in the memory first particular additional information relating to a usage of the medical examination device while correlating the first particular additional information with the identification, writing down second particular additional information relating to a circulation in the memory while correlating the second particular additional information with the identification, writing down third particular additional information relating to an inspection in the memory while correlating the third particular additional information with the identification, reading out one or more pieces from among the first to third particular additional information based on the identification, and sharing and utilizing plural pieces of particular additional information about the medical examination device among a plurality of users based on the identification.

An information processing method of another aspect according to the present invention that utilizes a medical examination device as a medium, which has been assigned a unique identification used for medical examinations and diagnoses, and a memory into which particular additional information about the medical examination device is remotely writable through a network based on the identification of the medical examination device includes the steps of identifying the identification of the medical examination device, and writing down in the memory first particular additional information relating to a usage of the medical examination device while correlating the first particular additional information with the identification, writing down second particular additional information relating to a circulation in the memory while correlating the second particular additional information with the identification, writing down third particular additional information relating to an inspection in the memory while correlating the third particular additional information with the identification, writing down fourth particular additional information relating to a disposal after the inspection in the memory while correlating the fourth particular additional information with the identification, reading out one or more pieces from the first to fourth particular additional information based on the identification, and sharing and utilizing plural pieces of particular additional information about the medical examination device among a plurality of users based on the identification.

An information processing method of another aspect according to the present invention that utilizes a medical examination device as a medium, which has been assigned a unique identification used for medical examinations and diagnoses, and a memory into which particular additional information about the medical examination device is remotely writable through a network based on the identification of the medical examination device includes the steps of identifying the identification of the medical examination device, and writing down in the memory first particular additional information relating to a usage of the medical examination device while correlating the first particular additional information with the identification, writing down second particular additional information relating to a circulation in the memory while correlating the second particular additional information with the identification, writing down, through an inspected person, third particular additional information relating to an inspection in the memory while correlating the third particular additional information with the identification, reading out one or more pieces from the first to third particular additional information based on the identification, and sharing and utilizing plural pieces of particular additional information about the medical examination device among a plurality of users based on the identification.

An information processing method that utilizes a medical examination device as a medium, which has been assigned a unique identification used for medical examinations and diagnoses, a memory into which particular additional information about the medical examination device is remotely writable through a network based on the identification of the medical examination device, a plurality of input / output units for remotely writing information into and reading the information from the memory through the network based on the identification of the medical examination device, includes the step of sharing and utilizing the particular additional information about the medical examination device among a plurality of users based on the identification.

In the above information processing method, the network may be the Internet. The particular additional information relating to a usage of the medical examination device may include information of a lifetime of the medical examination device. The medical examination device may be a device for inspection with a quarts crystal microbalance (QCM) reaction. The medical examination device may be a DNA chip. The medical examination device may be a lab on a chip that forms a channel on a substrate for processes on the substrate through a chemical or physical reaction. The medical examination device may be a protein chip. The medical examination device may be a DNA micro-array.

An information processing system of another aspect according to the present invention includes a medical examination device as a medium, which has been assigned a unique identification used for medical examinations and diagnoses, a memory into which particular additional information about the medical examination device is remotely writable through a network based on the identification of the medical examination device, and a plurality of input units for remotely writing the particular additional information down in the memory through the network based on the identification of the medical examination device, the input units being provided at least for a supplier of the medical examination device, a seller who sells the medical examination device supplied by the supplier, and an inspection institution that inspects the medical examination device.

An information processing system of another aspect according to the present invention includes a medical examination device as a medium, which has been assigned a unique identification used for medical examinations and diagnoses, a memory into which particular additional information about the medical examination device is remotely writable through a network based on the identification of the medical examination device, and a plurality of input units for remotely writing the particular additional information down in the memory through the network based on the identification of the medical examination device, the input units being provided at least for a supplier of the medical examination device, a seller who sells the medical examination device supplied by the supplier, and an examinee subject to an examination using the medical examination device.

An information processing system includes a medical examination device as a medium, which has been assigned a unique identification used for medical examinations and diagnoses, a memory, particular additional information about the medical examination device being remotely writable into and readable from the memory through a network based on the identification of the medical examination device, and a plurality of input / output units for remotely writing and reading the particular additional information in and from the memory through the network based on the identification of the medical examination device, wherein a plurality of users share and utilize, based on the identification, the particular additional information including the usage of the medical examination device which has been written while correlated with the identification in the memory.

In the above information processing system, the network may be the Internet. The particular additional information relating to a usage of the medical examination device may include information of a lifetime of the medical examination device. The medical examination device may be a device for inspection with a quarts crystal microbalance (QCM) reaction. The medical examination device may be a DNA chip. The medical examination device may be a lab on a chip that forms a channel on a substrate for processes on the substrate through a chemical or physical reaction. The medical examination device may be a protein chip. The medical examination device may be a DNA micro-array.

Other objects and further features of the present invention will become readily apparent from the following description of the following embodiments which are described by way of example only with reference to the accompanying drawings in which:
FIG. 1 is a view of a first embodiment according to the present invention showing one mode of share and utilization of a medical examination device among plural users, which has been shipped by a medical examination device vendor.
FIG. 2 is a flowchart of a utilization mode of the medical examination device in the first embodiment shown in FIG. 1.
FIG. 3 is a view of a second embodiment according to the present invention showing one mode of share and utilization of a medical examination device among plural users, which has been shipped by a medical examination device vendor.
FIG. 4 is a flowchart of a utilization mode of the medical examination device in the second embodiment shown in FIG. 3.
FIG. 5A is a view of a medical device using a DNA chip of one embodiment according to the present invention. FIG. 5B is a view of a medical device using a DNA chip of another embodiment according to the present invention.
FIG. 6 is a view of a first embodiment according to the present invention for explaining a system that uses a network with the Internet of the first embodiment according to the present invention to enable information to be written in and read from storage means in a server.
FIG. 7 is a view of a second embodiment according to the present invention for explaining a system that uses a LAN with the Internet of the first embodiment according to the present invention to enable information to be written in and read from storage means in a server.

When the above information processing method and system are applied, for example, an information processing method utilizes a MED such as a DNA chip, which has been assigned a unique identification used for medical examinations, diagnoses, etc. a memory in a server, etc. into which particular additional information about the medical examination device is remotely writable and readable through a network, such as the Internet, based on the identification of the medical examination device, and plural input / output units (such as PCs provided in an MED supplier, seller and inspection institution) for remotely writing particular additional information relating to a usage of the MED into and reading the information from the memory through the network. Thereby, the particular additional information may be shared and utilized among a plurality of users based on the identification.

This enables, for example, the MED vendor to manage a lifetime of the MED, as well as realizing real-time managements of the usage of the MED in a process of circulation and at a purchaser. The medical institution and drugstore may process an MED within its lifetime, and easily recognize the contents and usage of the MED and inform a user of the information.

Moreover, an individual user may easily recognize the usage of the MED. The inspection institution recognizes a use record of a MED, providing more accurate inspection and detection, and easily informing an inspection result of the MED. The medical institution and individual user may promptly and accurately recognize an inspection result by the inspection institution.

According to the above information processing method and system, sharing and utilizing of particular additional information among plural users based on the identification would effectively utilize the particular additional information of the MEDs. For example, the medical institution may recognize how the MED has been used. Here, it is conceivable to commit a disposal of any used MED to a medical waste disposal company without delivering the MED to the MED vendor for recycling. In this case, the company, etc. to whom the inspection institution has committed a disposal may register the disposal information relying upon the ID assigned to the MED. It is possible to inform medical institutions and independent users that MEDs, from which personal information of the independent users may be disclosed, have securely processed, for example, by uploading information of collected and disposed MEDs on a server on the network.

A description will now be given of embodiments according to the present invention.

### FIRST EMBODIMENT

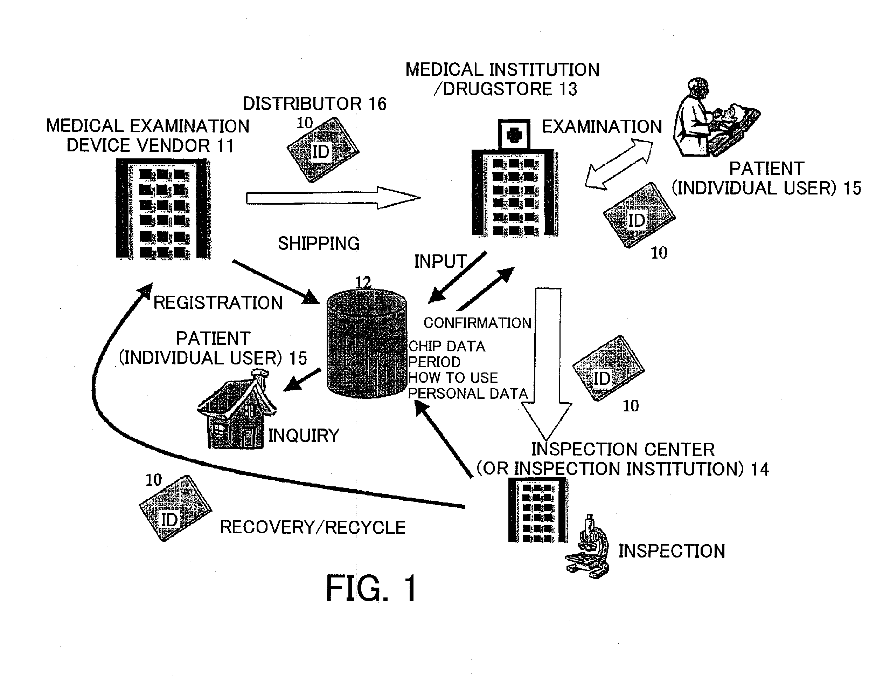

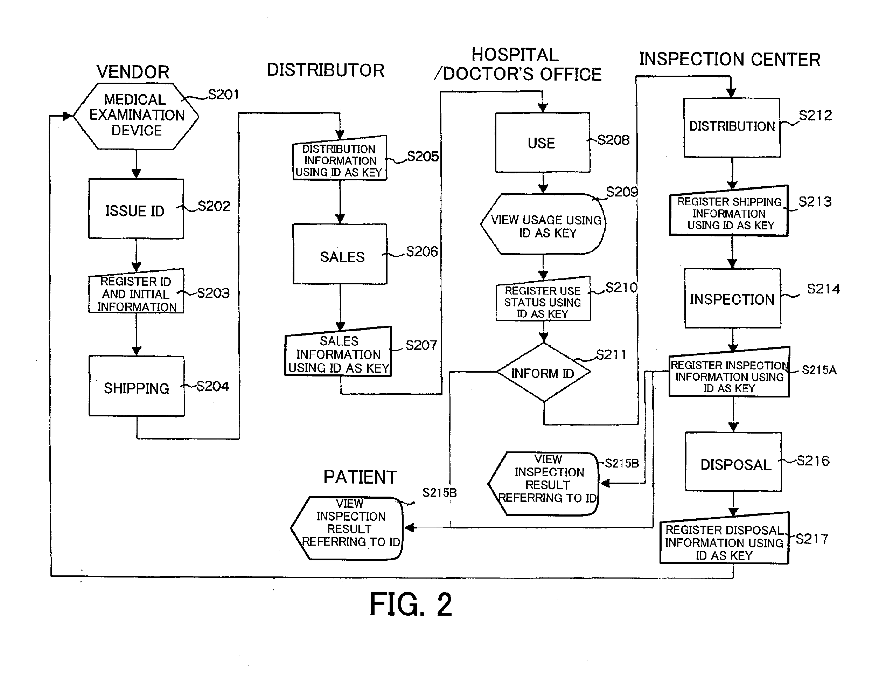

FIG. 1 is a view of a first embodiment according to the present invention showing one mode of share and utilization of an MED among plural users, which has been shipped by an MED vendor. FIG. 2 is a flowchart of a utilization mode of the MED in the first embodiment shown in FIG. 1. Here, a term "MED" generalizes such instruments as a DNA chip, Lab on a Chip, a micro-array, and a micro-TAS, which are used for medical diagnoses.

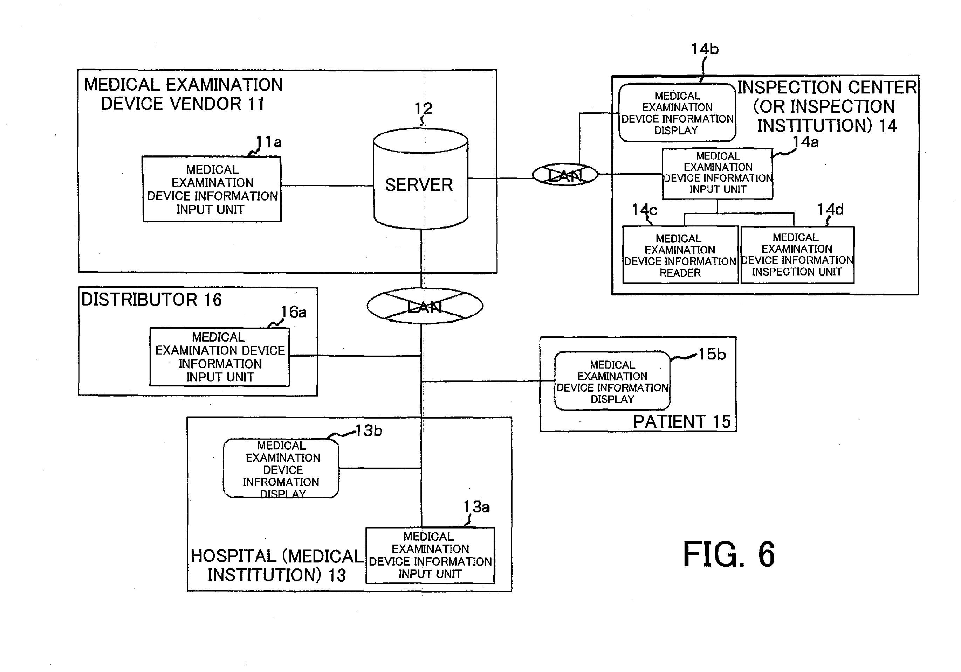

A description will now be given of a first embodiment according to the present invention using flowcharts shown in FIGs. 1 and 2, and a system explanatory view in FIG. 6. A description will now be given of a system with reference to FIG. 6.

The MED vendor 11 that makes MEDs 10 serves to built a system and manage a server 12 as storage means in this system as well as having an MED information input unit, such as a personal computer ("PC") connected to the server 12.

The distributor 12 who sells the MEDs 10 may input information through the Internet via the server 12 and a LAN, and has an MED information input unit 16a, such as a PC.

A medical institution 13, such as a hospital, may input and output information through the Internet via the server 12 and a LAN, and includes an MED information input unit 13a and an MED information display 13b, such as a PC.

An inspection center 14 which inspects the MEDs 10 may input and output information through the Internet via the server 12 and a LAN, and includes an MED information input unit 14a and an MED information display 14b, such as a PC. In addition, the inspection center 14 includes a MED result reader 14c and MED inspection unit 14d for inspecting the MEDs 10.

A patient or individual user 15 may input information through the Internet via the server 12 and the LAN, and possesses a medical inspection information display 15b, such as a PC.

A description will be given of a system flow.

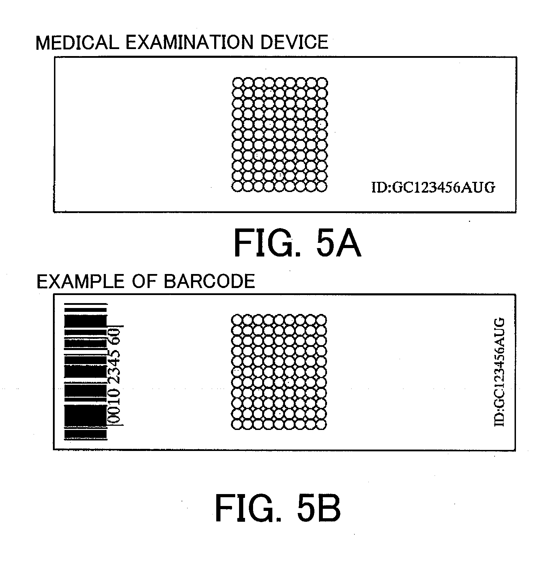

The MED vendor 11 makes the MED 10 (step S201), and assigns an ID to the MED 10 (step S202). Here, a medical device, such as a DNA chip shown in FIG. 5, is made as the MED 10, and assigned a symbol that represents an ID as shown in FIG. 5A, or a barcode as shown in FIG. 5B.

The MED vendor 11 registers the ID assigned to the MED 10 in the server 12 (step S203) as well as its usage and usable term or lifetime unique to this MED (step S203). In the DNA chips, etc., actual usage for a patient changes according to novel developments, and a registration of the usage is extremely important information for future inspection purposes.

After this registration, the MED vendor 11 ships the MED 10 (step S204).

The distributor 16 that handles circulations and distributions of the MEDs 10 stores the MEDs 10 for sale. The MED vendor 11 may serve as the distributor 16. The distributor 16 registers information on a process of circulation, such as temperature control, room temperature exposure time, and couriers and dates, with IDs as keys in the server 12 (step 205). The distributor 16 sells the MEDs 10 (step S206), and registers information of purchasers and sales dates with IDs as keys in the server 12.

The medical institution 13, such as a hospital and a clinic, uses purchased MEDs 10 (step S208), while using an ID as a key to view reference information necessary for the MED 10 as MED-related information which the MED vendor 11 has already registered, such as usage and expiration date of the MED 10 (step S209), thereby reducing misapplications with the utmost care. The medical institution 13 registers a use status and a user or patient name in the server (step S210), and informs the user or patient 15 of the ID of the MED 10 (step S211).

The medical institution 13, etc. that has used the MED 10 disposes the MEDs 10 by itself, or delivers the MEDs 10 to an inspection center, such as a specialized evaluation institution that undertakes depositary inspections of MEDs 10 (step S212). The inspection center or institution 14 that receives the delivery uses an ID as a key to register a delivery statue and condition, such as a shipping state, temperature control state, a time period and the number of days required, and a reception date in the server 12 (step S213).

The inspection center 14 inspects the MED 10 (step S214), and registers the inspection information in the server 12 (step S215A). The inspection center 14 disposes the useless MED 10 (step S216), and registers the disposal state, method, date and time, etc., in the server 12 (step S217).

Alternatively, the inspection center 14 delivers the MEDs 10 to the MED vendor 11 for recycling without disposal. The MED vendor 11 determines whether the MED 10 is to be recycled, by taking into consideration its lifetime (such as the number of usable times, and the number of elapsed days from the manufacture date) of the MED 10, and management information (such as temperature control and delivery state) in a process of circulation at the medical institution and inspection center, etc. The record information was read out from the server 12 using an ID as a key. Of course, a new ID is assigned in recycling. However, a correlation with a prior ID is registered in the server 12 so that all the records after manufacture may be tracked in determining recycling again.

The medical institution 13 (such as a doctor) may use an ID as a key to view an inspection result of the MED 10 registered by the inspection center 14 (step S215B). The patient 15 is restricted to access the server 12 so that he may access the MED 10 that was used for his own examination and view its inspection result. He cannot view information inappropriate to be informed to him, such as information other than his medical information, even if the MED 10 was used for his examination.

The above embodiment uses a system etc. that uses a network, e.g., LAN, using the Internet shown in FIG. 6 to enable the MED vendor 11, the distributor 16, the inspection center 14, etc. to write such information about the MEDs 10 as an ID, usage, lifetime, information on a process of circulation, and inspection information, down in the storage means in the server 2. Thereby, it uses an ID of the MED 10 as a key to remotely write particular additional information in the server 12 through input means provided in these MED vendor 11, distributor 16, inspection center 14, etc. via the network.

In this way, a manufacturer of the MEDs and a plurality of users, such as a computer of the MED vendor 11, a computer of a doctor or medical institution 13 as a user, a computer of the individual 15, and a computer of the inspection center 14, register their statuses in the server 12 on the network, enabling a unitary management of all the information relating to the MED 10 using a chip ID. When the MED vendor 11 builds this system, it restricts accesses to viewable information based on a status of each client, checks any unfilled necessary information, and manages stored or registered record information, such as a date and a writer.

This system enables an ID of one MED to be commonly used for a plurality of cites, and a plurality of users to use the ID as a key to register, view and utilize information of the MED 10. Thereby, the MED vender 11 may manage an expiration date and use status of each shipped MED 10, enlighten appropriate use, and recognize whether it is properly used. The distributor 16 or a MED seller may readily recognize a shipping status from the MED vendor 11. Each user may recognize a status and content of the MED 10.

The contents registered by the inspection center 14 become viewable on the network. Thus, a user may easily obtain an inspection result without going to the medical institution directly or waiting for a notice to be sent by mail etc. The inspection center 14 may recognize how long and how the MED 10 has been used, a method of properly using the MED 10, and its expiration date, and this function provides a definite inspection. Each user may recognize that the MED that stores his personal information has been definitely processed through registrations of a collection and recycling by the MED vendor 11.

### SECOND EMBODIMENT

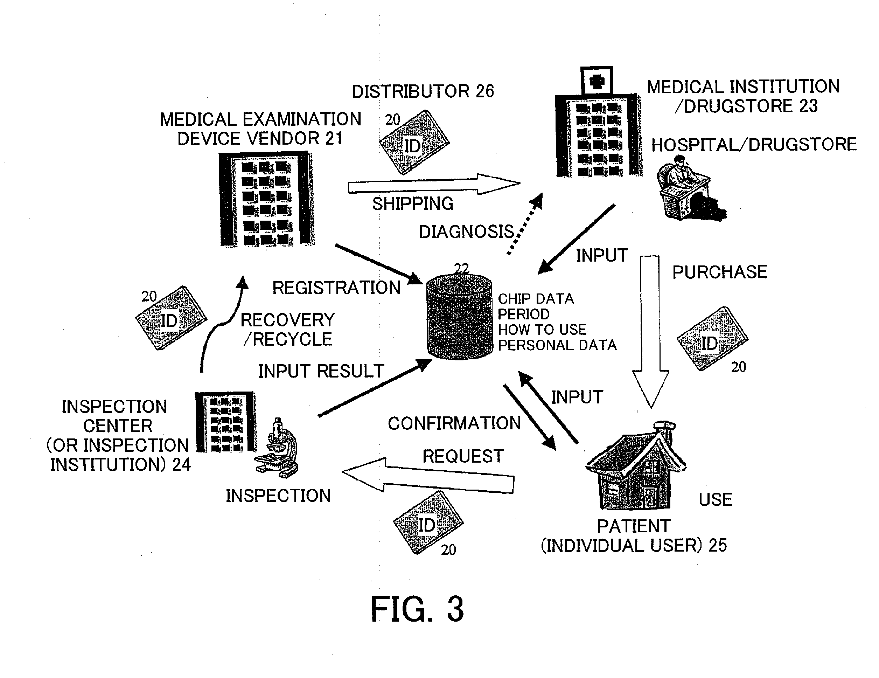

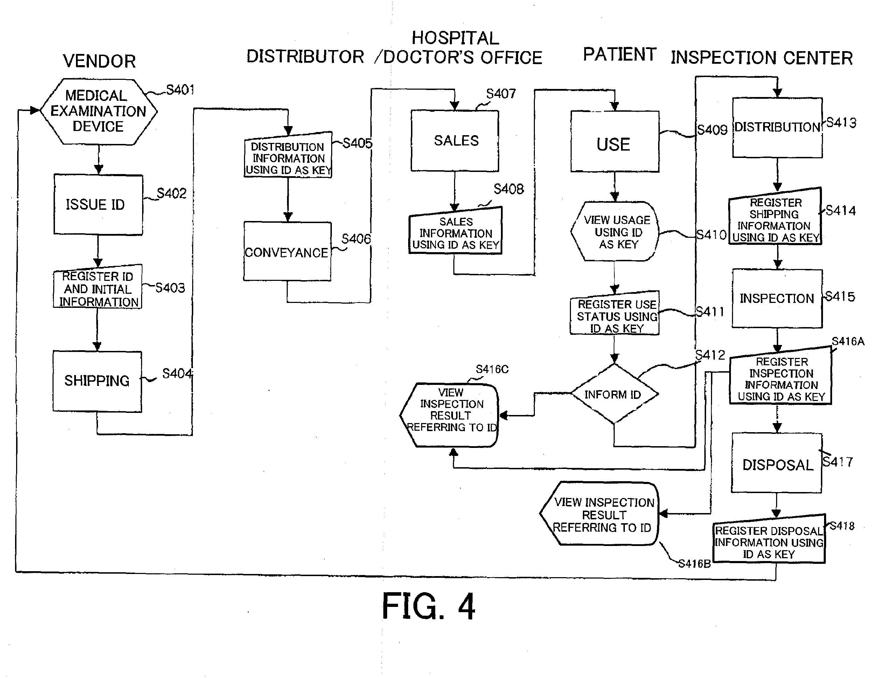

FIG. 3 is a view of a second embodiment according to the present invention showing another mode of share and utilization of a MED 20 among plural users, which has been shipped by a MED vendor 21. FIG. 4 is a flowchart of a utilization mode of the MED 20 in the second embodiment shown in FIG. 3. This embodiment may also use the same MED as that used for the first embodiment for the MED 20.

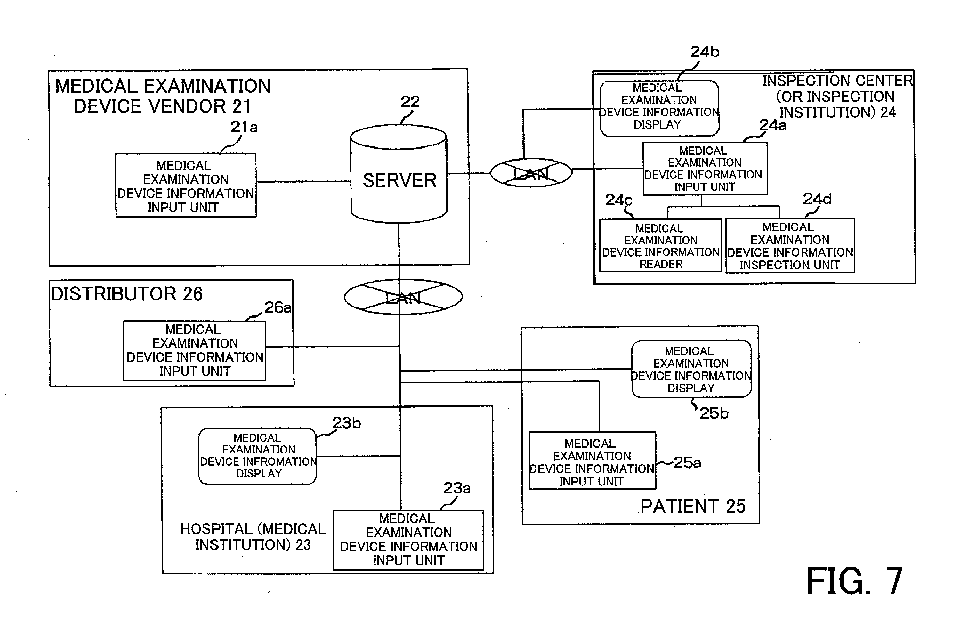

A description will now be given of a first embodiment according to the present invention using flowcharts shown in FIGs. 3 and 4, and a system explanatory view in FIG. 7. A description will be omitted for those elements which are the same as those in the first embodiment. A description will now be given of a system with reference to FIG. 7.

The MED vendor 21 that makes MEDs 20 serves to built a system and manage a server 22 as storage means in the system as well as having an MED information input unit, such as a PC connected to the server 22.

A distributor 22 who distributes to sell the MEDs 20 may input information through the Internet via the server 22 and a LAN, and has an MED information input unit 26a, such as a PC.

A medical institution 23, such as a hospital, may input and output information through the Internet via the server 22 and a LAN, and has an MED information input unit 23a and an MED information display 23b, such as a PC.

An inspection center 14 which inspect the MEDs 20 may input and output information through the Internet via the server 22 and a LAN, and has an MED information input unit 24a and an MED information display 24b, such as a PC. In addition, the inspection center 24 has a MED result reader 24c and MED inspection unit 24d for inspecting the MEDs 10.

The patient (or individual user) 25 is configured to input information through the Internet via the server 22 and the LAN, and has MED information input unit 25a and medical inspection information display 25b, such as a PC.

A description will be given of a system flow.

The MED vendor 21 makes the MED 20 (step S401), and assigns an ID to the MED 20 (step S202). The MED vendor 21 registers the ID assigned to the MED 20 in the server 22 (step S403) as well as usage and use term (or lifetime) unique to this MED 20 (step S403). After this registration, the MED vendor 21 ships the MED 20 (step S404).

The distributor 26 stores the shipped MEDs 20 for delivery. The MED vendor 21 may serve as the distributor 26. The distributor 26 registers information on a process of circulation, such as temperature control, room temperature exposure time, and shipping agents and dates, with IDs as keys in the server 22 (step 405). The distributor delivers the MEDs 20 to the medical institution 23, such as a hospital and clinic (step S406).

The medical institution 13 as a purchaser, such as a hospital and a clinic, directly sells MEDs 20 (step S407), and registers information, such as a sales date, a purchaser (or a name of a patient), in the server 22 using an ID as a key (step S408). The patient (or individual user) 25 himself uses the MED 20 sold from the medical institution 23 as the above purchaser (step S409). Prior to the use, he retrieves usage and expiration date of the MED 20 using the ID as a key and views reference information for use with the MED 20 (step S410). The patient (or individual user) 25 uses the ID as a key to register a use status and date in the server (step S411), and informs the medical institution (or doctor of a hospital or clinic) 23 of the ID of the used MED 20 (step S412). A system that serves as a specific informing means registers the notification in the server 22 using the ID as a key in the server 22, and informs the medical institution 23 of the information.

The patient (or individual user) 25 delivers the MED 20 to the inspection center or institution 24 (step S413). The inspection center or institution 24 that receives the delivery uses an ID as a key to register a delivery statue and condition, such as a shipping state, temperature control state, and a time period and the number of days required, in the server 12. The inspection center 24 inspects the MED 20 (step S415), and registers the inspection result in the server 22 (step S216A). The inspection center 24 disposes the useless MED 20 (step S417), and registers the disposal state, method, date and time, etc., in the server 22 (step S418).

Alternatively, the inspection center 24 delivers the MEDs 20 without disposal to the MED vendor 21 for recycling (step S418). The MED vendor 21 recycles the MED 20 by processing the MED 20 so as to enable it to be recycled, and registering in the server 22 whether individual information, such as patient's personal information (or genetic information), has been properly processed. The process relating to recycling is similar to the first embodiment.

The medical institution 23 (such as a doctor) may use an ID as a key to view the inspection result of the MED 20 registered by the inspection center 24 (step S416B). The patient 25 may also view the inspection result of the MED 20 registered by the inspection center 24 using the informed ID as a key (step S416C). The patient 25 may also confirm that the MED 20 has been properly processed.

The above embodiment uses a system etc. that uses a network, e.g., LAN, using the Internet shown in FIG. 7 to enable the MED vendor 21, the distributor 26, the patient (or individual user) 25, etc. to write such information about the MEDs 20 as an ID, usage, lifetime, information on a process of circulation, and inspection information, down in the storage means in the server 22. Thereby, it uses an ID of the MED 20 as a key to remotely write particular additional information in the server 22 through input means provided in these MED vendor 21, distributor 26, patient (or individual user) 25, etc. via the network.

In this way, the MED vendor 11, the distributor 26, the patient (or individual user) 25, and the inspection center 24 share information using an ID of the MED as a key which has been registered in the server 22 on the network. This system enables an ID of one MED to be commonly used for a plurality of cites, and a plurality of users to use the ID as a key to register, view and utilize information of the MED 20. Thereby, the MED vendor 21 may manage an expiration date and use status of each shipped MED 20, and the seller may readily recognize a shipping status from the MED vendor 21. Each patient (or individual user) 25 may recognize a status and content of the MED 20 to be used. The contents registered by the inspection center 24 become viewable on the network. Moreover, the patient (or individual user) 25 may share information with a doctor of the medical institution 23 for easy inspection.

Since the information is readily shared with the medical institution 23, a prompt care would be expected in abnormal time. The inspection center 24 may recognize how long and how the MED 20 has been used, and this function is useful for a careful inspection. Each patient (or individual user) may recognize that the MED that stores his personal information has been securely processed through registrations of a collection and recycling of the MED vendor 21.

As many apparently widely different embodiments of the present invention can be made without departing from the scope thereof, it is to be understood that the invention is not limited to the specific embodiments thereof except as defined in the claims.

## Claims

1. An information processing method that utilizes a medical examination device as a medium, which has been assigned a unique identification used for medical examinations and diagnoses, and a memory into which particular additional information about the medical examination device is remotely writable through a network based on the identification of the medical examination device, said method comprising the steps of:
identifying the identification of the medical examination device, and writing down in the memory the particular additional information relating to a usage of the medical examination device while correlating the particular additional information with the identification; and
sharing and utilizing the particular additional information about the medical examination device among a plurality of users based on the identification.

2. An information processing method that utilizes a medical examination device as a medium, which has been assigned a unique identification used for medical examinations and diagnoses, and a memory into which particular additional information about the medical examination device is remotely writable through a network based on the identification of the medical examination device, said method comprising the steps of:
identifying the identification of the medical examination device, and writing down in the memory first particular additional information relating to a usage of the medical examination device while correlating the first particular additional information with the identification;
writing down second particular additional information in the memory while correlating the second particular additional information with the identification;
reading out one or more pieces from among the first and second particular additional information based on the identification; and
sharing and utilizing plural pieces of particular additional information about the medical examination device among a plurality of users based on the identification.

3. An information processing method that utilizes a medical examination device as a medium, which has been assigned a unique identification used for medical examinations and diagnoses, and a memory into which particular additional information about the medical examination device is remotely writable through a network based on the identification of the medical examination device, said method comprising the steps of:
identifying the identification of the medical examination device, and writing down in the memory first particular additional information relating to a usage of the medical examination device while correlating the first particular additional information with the identification;
writing down second particular additional information relating to an inspection in the memory while correlating the second particular additional information with the identification;
writing down third particular additional information in the memory while correlating the third particular additional information with the identification;
reading out one or more pieces from among the first to third particular additional information based on the identification; and
sharing and utilizing plural pieces of particular additional information about the medical examination device among a plurality of users based on the identification.

4. An information processing method that utilizes a medical examination device as a medium, which has been assigned a unique identification used for medical examinations and diagnoses, and a memory into which particular additional information about the medical examination device is remotely writable through a network based on the identification of the medical examination device, said method comprising the steps of:
identifying the identification of the medical examination device, and writing down in the memory first particular additional information relating to a usage of the medical examination device while correlating the first particular additional information with the identification;
writing down second particular additional information relating to a circulation in the memory while correlating the second particular additional information with the identification;
writing down third particular additional information relating to an inspection in the memory while correlating the third particular additional information with the identification;
reading out one or more pieces from among the first to third particular additional information based on the identification; and
sharing and utilizing plural pieces of particular additional information about the medical examination device among a plurality of users based on the identification.

5. An information processing method that utilizes a medical examination device as a medium, which has been assigned a unique identification used for medical examinations and diagnoses, and a memory into which particular additional information about the medical examination device is remotely writable through a network based on the identification of the medical examination device, said method comprising the steps of:
identifying the identification of the medical examination device, and writing down in the memory first particular additional information relating to a usage of the medical examination device while correlating the first particular additional information with the identification;
writing down second particular additional information relating to a circulation in the memory while correlating the second particular additional information with the identification;
writing down third particular additional information relating to an inspection in the memory while correlating the third particular additional information with the identification;
writing down fourth particular additional information relating to a disposal after the inspection in the memory while correlating the fourth particular additional information with the identification;
reading out one or more pieces from the first to fourth particular additional information based on the identification; and
sharing and utilizing plural pieces of particular additional information about the medical examination device among a plurality of users based on the identification.

6. An information processing method that utilizes a medical examination device as a medium, which has been assigned a unique identification used for medical examinations and diagnoses, and a memory into which particular additional information about the medical examination device is remotely writable through a network based on the identification of the medical examination device, said method comprising the steps of:
identifying the identification of the medical examination device, and writing down in the memory first particular additional information relating to a usage of the medical examination device while correlating the first particular additional information with the identification;
writing down second particular additional information relating to a circulation in the memory while correlating the second particular additional information with the identification;
writing down, through an inspected person, third particular additional information relating to an inspection in the memory while correlating the third particular additional information with the identification;
reading out one or more pieces from the first to third particular additional information based on the identification; and
sharing and utilizing plural pieces of particular additional information about the medical examination device among a plurality of users based on the identification.

7. An information processing method that utilizes a medical examination device as a medium, which has been assigned a unique identification used for medical examinations and diagnoses, a memory into which particular additional information about the medical examination device is remotely writable through a network based on the identification of the medical examination device, a plurality of input / output units for remotely writing information into and reading the information from the memory through the network based on the identification of the medical examination device, said method comprising the step of sharing and utilizing the particular additional information about the medical examination device among a plurality of users based on the identification.

8. A method according to any one of claims 1 through 7, wherein the network is the Internet.

9. A method according to any one of claims 1 through 7, wherein the particular additional information relating to a usage of the medical examination device includes information of a lifetime of the medical examination device.

10. A method according to any one of claims 1 through 7, wherein the medical examination device is a device for inspection with a quarts crystal microbalance (QCM) reaction.

11. A method according to any one of claims 1 through 7, wherein the medical examination device is a DNA chip.

12. A method according to any one of claims 1 through 7, wherein the medical examination device is a lab on a chip that forms a channel on a substrate for processes on the substrate through a chemical or physical reaction.

13. A method according to any one of claims 1 through 7, wherein the medical examination device is a protein chip.

14. A method according to any on of claims 1 through 7, wherein the medical examination device is a DNA micro-array.

15. An information processing system comprising:
a medical examination device as a medium, which has been assigned a unique identification used for medical examinations and diagnoses;
a memory into which particular additional information about the medical examination device is remotely writable through a network based on the identification of the medical examination device; and
a plurality of input units for remotely writing the particular additional information down in the memory through the network based on the identification of the medical examination device, said input units being provided at least for a supplier of the medical examination device, a seller who sells the medical examination device supplied by the supplier, and an inspection institution that inspects the medical examination device.

16. An information processing system comprising:
a medical examination device as a medium, which has been assigned a unique identification used for medical examinations and diagnoses;
a memory into which particular additional information about the medical examination device is remotely writable through a network based on the identification of the medical examination device; and
a plurality of input units for remotely writing the particular additional information down in the memory through the network based on the identification of the medical examination device, said input units being provided at least for a supplier of the medical examination device, a seller who sells the medical examination device supplied by the supplier, and an examinee subject to an examination using the medical examination device.

17. An information processing system comprising:
a medical examination device as a medium, which has been assigned a unique identification used for medical examinations and diagnoses;
a memory, particular additional information about the medical examination device being remotely writable into and readable from the memory through a network based on the identification of the medical examination device; and
a plurality of input / output units for remotely writing and reading the particular additional information in and from the memory through the network based on the identification of the medical examination device, wherein a plurality of users share and utilize, based on the identification, the particular additional information including the usage of the medical examination device which has been written while correlated with the identification in the memory.

18. A system according to any one of claims 15 through 17, wherein the network is the Internet.

19. A system according to any one of claims 15 through 17, wherein the particular additional information relating to a usage of the medical examination device includes information of a lifetime of the medical examination device.

20. A system according to any one of claims 15 through 17, wherein the medical examination device is a device for inspection with a quarts crystal microbalance reaction.

21. A system according to any one of claims 15 through 17, wherein the medical examination device is a DN_{A} chip.

22. A system according to any one of claims 15 through 17, wherein the medical examination device is a lab on a chip that provides a channel on a substrate for processes on the substrate through a chemical or physical reaction.

23. A system according to any one of claims 15 through 17, wherein the medical examination device is a protein chip.

24. A system according to any one of claims 15 through 17, wherein the medical examination device is a DNA micro-array.
